# EUROPEAN PATENT APPLICATION

(11) **EP 1 405 602 A1**
(43) Date of publication of application: **07.04.2004**
(21) Application number: 03077802.1
(22) Date of filing: 08.09.2003
(51) Int. Cl.: A61B 17/16, A61B 17/32

(54) **Instrument for removing and collecting flakes or shavings from bones by scraping**

(30) Priority: 02.10.2002 IT RE20020075
(71) Applicant: C.G.M. S.P.A., 42015 Correggio (Reggio Emilia) (IT)
(72) Inventor: Parmigiani, Corrado Saverio, 42015 Correggio (Reggio Emilia) (IT)
(74) Representative: Corradini, Corrado

(57) **Abstract**

The instrument for removing and collecting flakes or shavings from bones by scraping comprises a handle carrying a scraper blade (50) positioned at its front end, and possesses in its front end portion a collection chamber (R) for the removed material; the instrument is characterised in that the handle comprises: an elongate inner element (20), having at least a front portion (21) with a cylindrical surface; a tubular outer casing (30) which encloses the inner element (20) about its entire perimeter and conforms closely to the outer surface of its front portion (21); a longitudinal rod (40) having a cross-section less than the cross-section of the collection chamber (R) to extend longitudinally along and within the chamber, its rear end being fixed to the inner element (20); the scraper blade (50) being fixed to the front end of the rod (40). An object of the instrument is to make its cross-section as small as possible in order to render the withdrawal technique only minimally invasive.

## Description

This invention relates to methods for the reconstructive and regenerative removal of bone tissues in oro-maxillo-facial, plastic, periodontal and implantological orthopedic surgery together with plastic surgery techniques on bone.

In the recent past, instruments have been developed for withdrawing blocks or cylinders of bone from various regions of the skeletal structure and for working and manipulating such blocks with the purpose of obtaining granules or particles of dimensions suitable for biological regenerative tissue requirements.

These instruments have resulted in a growth of autologous bone withdrawal techniques and processing of the withdrawn bone material, enabling bone granules to be obtained for filling bone defects or for augmenting skeletal structures; these procedures reduce the cost of surgery compared with other synthetic or alloplastic products such as hydroxyapatite granules, processed coral, processed bone of heterologous origin, and demineralised human bank bone; autologous bone grafts also reduce the risk of virus and infection transmission while maintaining the vital functions of the withdrawn bone and the basic cells for stimulating and accelerating the reparation and reconstructive processes on hard and soft tissues.

To simplify and accelerate withdrawal, collection and trituration procedures while reducing post-operative patient discomfort, withdrawal methodologies have been refined in recent years by the introduction to the market of instruments for removing and collecting flakes or shavings from bone by scraping, these comprising a handle having a scraper blade positioned at the front end of the handle, and a collection chamber for removed material provided in their front end.

Said devices enable surface bone corticals to be removed by a special scraper blade provided on the instrument to generate thin bone flakes or shavings which are directly collected within the collection chamber.

The advanced technology of the scraper blade enables an excellent controllable cut to be obtained with light pressure. The bone flakes are collected via a slit located at the base of the blade to convey them into the collection chamber defined by a suitable protected vessel for their temporary preservation.

During their collection, the bone flakes are mixed with blood to form a concentrate of high bone density, ideal as a filler in regenerative techniques.

A simple sliding operation opens the collection chamber containing the previously removed material, making it immediately available for deposition within the receiving site in a rapid and secure manner, so eliminating further manipulations.

One of said instruments is described in US 5,683,406, which comprises a cutting edge positioned on the front side of a half moon-shaped aperture provide at the end of a slidable plate which closes the cavity of a handle also acting as the collector for the shavings.

The cutting edge of said half moon-shaped blade emerges on that side of the plate which is external to said cavity, and remains accessible from the outside with the blade in any position.

The handle cavity is closed by a slidable plate which exposes the shavings withdrawal aperture by bringing said cutting edge into contact with the donor bone and making it slide thereon while maintaining it pressed with a certain force.

This type of instrument is unable to be constructed with a cross-section smaller than a maximum dimension of about 6-4 mm. Moreover the pressure with which the cutting edge acts is determined essentially by the surgeon, with the risk that excessive pressure produces undesirable serious injuries to blood vessels or, more dangerously, to nerves or tendons.

An object of the present invention is to improve this type of instrument with the aim of enabling its cross-section to be made as small as possible, so that the withdrawal procedure becomes only minimally invasive.

Another object is to form, by the scraping action, shavings of smaller size on the basis of the applicational requirements of the surgeon in cases of bone reconstruction of minor defects, for example infraosseous tooth defects of periodontal type, so aiding the repair mechanisms of those specific narrow areas.

Another object is to provide an instrument the action of which is better controllable by the operator.

A further sector of utilization of the present invention is in bone plastic surgery, in which an osteotome is currently used for bone correction or corrective removal. This instrument is currently available for various surgical disciplines: dental, maxillo, orthopedics, etc. The manufacturing material is steel of various hardnesses, hence resterilizable; wear occurs after a number of times of use, but it can be resharpened by stone known as surgical stone.

It is used in particular in nose surgery for removing humps and/or bone projections, including side projections of the osseous septum using a hammer. In such cases there is a danger of poor precision and risky control of the instrument by the operator in endoscopic tunnel techniques. The patient can therefore suffer considerable damage due to hammer blows, resulting in edema (swelling) and ecchymosis (bruising) after the operation.

As an alternative, rhynoplasty often uses bone removal instruments known as files, also of resterilizable steel as in the case of the osteotome, and already used in various surgical disciplines. These present however more or less invasive dimensions, and teeth which effect a gradual removal over the entire area worked by the file and hence not perfectly defined.

With reference to this type of instrument, another object of the invention is to provide an instrument able to better control the removal of shavings on the basis of the aesthetic requirements of the tissues.

A further object is, in manufacturing the instrument, to be able to use commercially available components obtained using the most advanced constructional techniques, and which are already used for other uses and with other instruments or apparatus.

These and further objects are attained by the invention as characterised in the claims.

Generally, the handle of the instrument comprises:
an elongate inner element, of which at least a front portion has a cylindrical surface,
a tubular outer casing which encloses the inner element about its entire perimeter and adheres to the outer surface of its front portion,
said outer casing being slidable in a longitudinal direction relative to the inner element between a front position in which its front end portion projects forwards longitudinally to a maximum extent from the front portion of the inner element, to define said collection chamber, and a rear position in which the outer casing is displaced rearwards to project from the inner element to a lesser extent than in the front position, the collection chamber being at least partly open,
a longitudinal rod having a cross-section less than the cross-section of the collection chamber to extend longitudinally along and within the chamber, it being enclosed by the outer casing and having its rear end fixed to the inner element,
the scraper blade being fixed to the front end of the longitudinal rod and disposed to the front of the front end portion of the casing.

The scraper blade and the longitudinal rod are independent of the outer casing, at least part of the rod being spaced in a radial direction from the inner surface of the outer casing so that it can flex towards said surface in a radial direction when the blade, when in use, is urged against the bone in a radial direction.

The longitudinal rod is of such dimensions and shape, in relation to its composition material, as to make it elastically deformable so that it moves in a direction radial to the front edge of the outer casing when the blade, when in use, is urged against the bone in a radial direction.

By virtue of the present invention, surface cortical bone can be withdrawn in narrow areas, in particular by intratissue tunnelling, for example in various structures of the orofacial system, with the object of considerably reducing post-operative discomfort to the patient and accelerating removal techniques. In this respect, when in use the instrument requires only a single small incision, of about 5-6 mm, to enable penetration between soft and hard tissues and hence minimize bleeding and incision scars. Moreover, by virtue of the new micro-blade, the size of the bone shaving is more suitable for treating bone defects in narrow spaces.

The invention is described in detail hereinafter with the aid of the accompanying figures which illustrate non-exclusive embodiments thereof by way of example.

Figure 1 is a perspective view of a first embodiment of the invention.

Figure 2 is a section on the vertical axial plane of Figure 1.

Figure 2A is the same section as Figure 2, but with the chamber R in the uncovered position.

Figure 2B is a plan view of Figure 2 from above.

Figure 3 is an enlarged detail of Figure 2.

Figure 4 is an enlarged detail of Figure 3 with the instrument in use.

Figure 5 is a front view of Figure 4.

Figure 6 is a perspective view of a second embodiment of the invention.

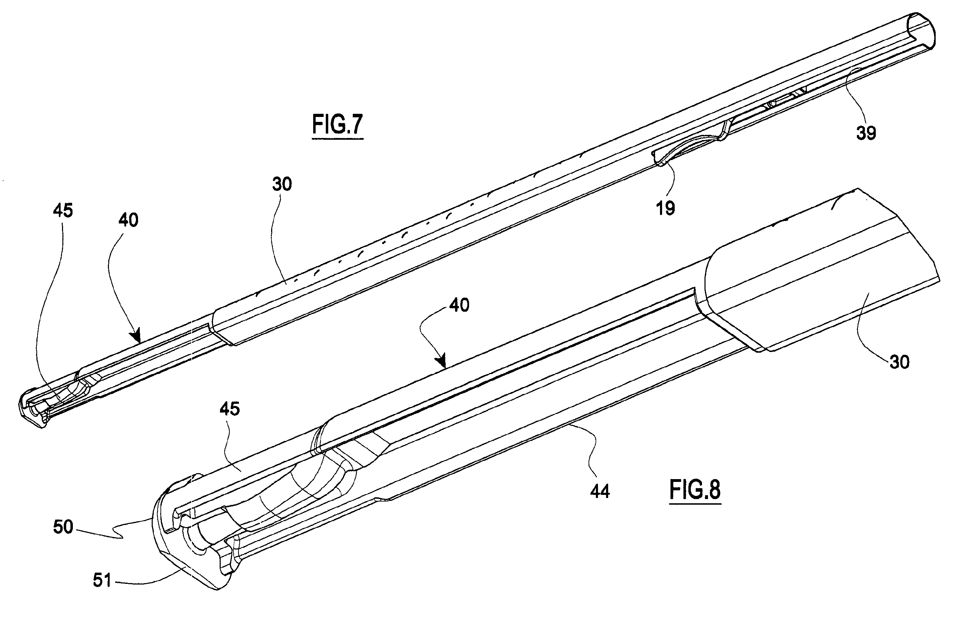

Figure 7 is a perspective view of Figure 6 from below with the chamber R in the uncovered position.

Figure 8 is an enlarged detail of Figure 7.

Figure 9 is a section on the vertical axial plane of Figure 6.

Figure 9A is the same section as Figure 9, but with the chamber R in the uncovered position.

Figure 10 is an enlarged detail of Figure 9.

Figure 11 is an enlarged detail of Figure 10 with the instrument in use.

Figure 12 is a section on the plane XII-XII of Figure 10.

Figure 13 is a front view (in the direction of the arrow (XIII) of Figure 11.

The instrument illustrated in the figures comprises a narrow elongate handle M to be gripped by the user's hand, it having a rear handgrip 10 and an elongate inner element 20.

The inner element 20 is connected coaxially at its rear to the handgrip 10, such as to form a single body M therewith, and possesses at least one front portion, specifically the front end portion, having a cylindrical surface (the term "cylindrical" meaning that its cross-section can be circular or have any other closed form).

About the inner element 20 there is a tubular outer casing 30 which encloses the inner element 20 along its entire outer perimeter to adhere both to the outer surface of its front end portion 21 and to other points of the rear portion, so as to be properly guided slidingly relative to the element 20 and adhere overall to it without slack.

The outer casing 30 is coupled to the inner element 20 such that it can slide in a longitudinal direction relative thereto but cannot rotate relative thereto about its longitudinal axis.

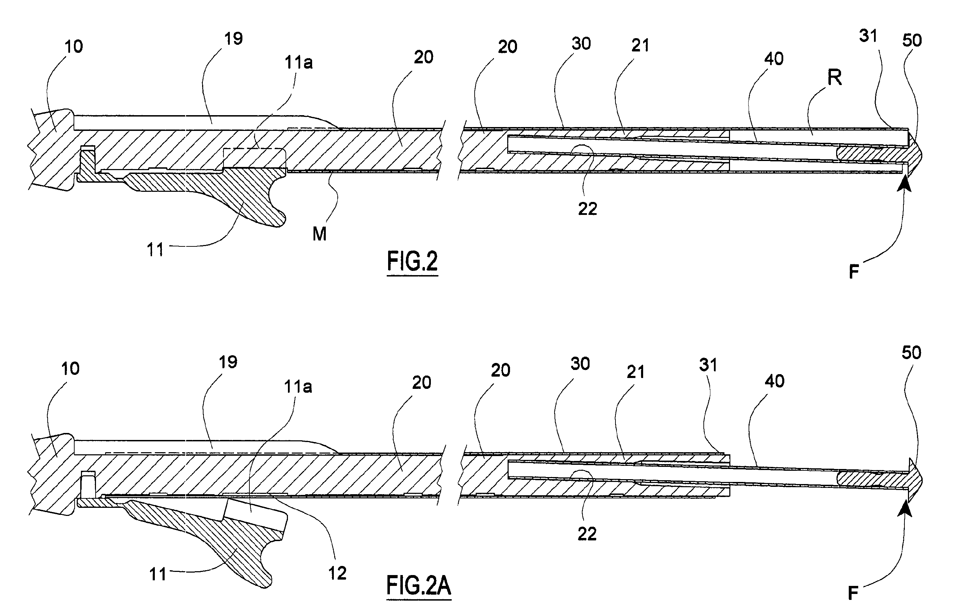

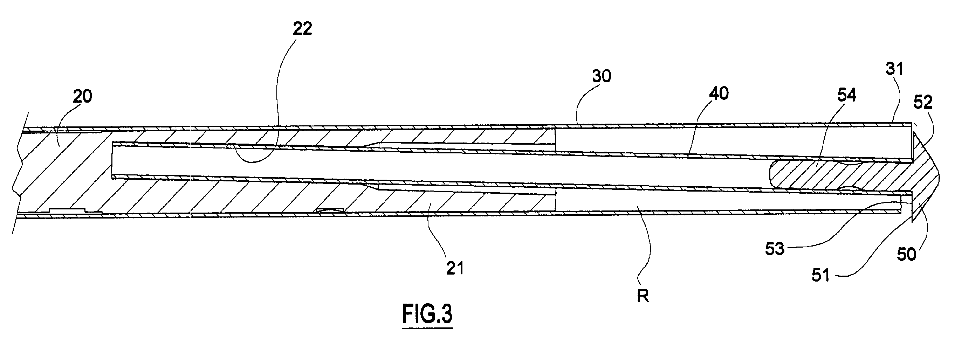

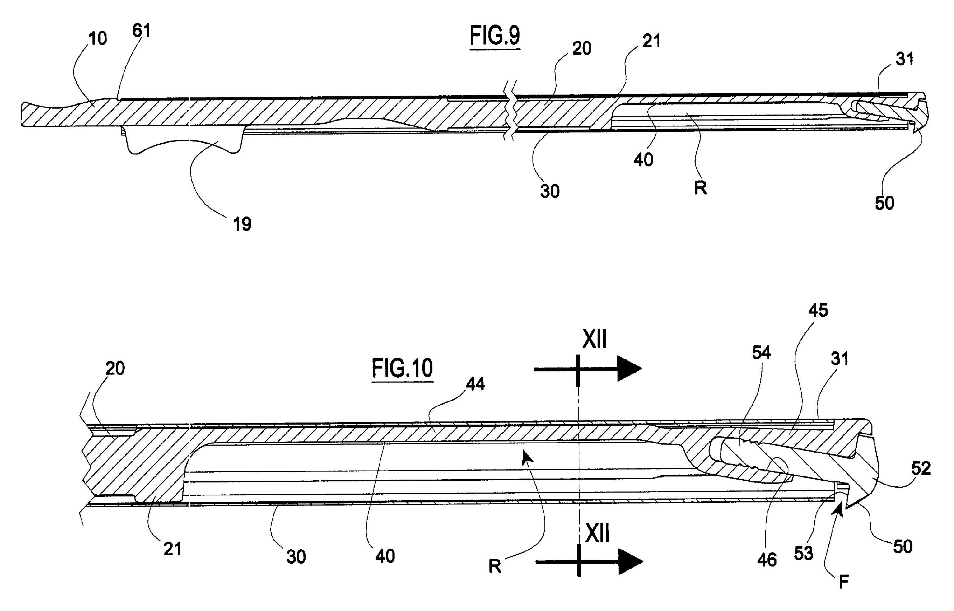

The outer casing 30 can slide in a longitudinal direction relative to the element 20 between a front position and a rear position. When in the front position (see Figures 3 and 10), its front end portion 31 projects forwards longitudinally to the maximum extent beyond the front end portion 21 of the inner element, to define a collection chamber R which is closed laterally by the casing 30, at its rear by the front of the front end portion 21 (to which the outer casing 30 adheres without slack) and at its front by the blade 50. When the outer casing 30 is pulled rearwards, it moves into a rear position in which it projects to a lesser extent beyond the inner element 20 than when in its front position, the collection chamber R then being at least partially opened by the outer casing 30 (see Figure 2A).

The rear handgrip 10 remains uncovered by the outer casing 30 and can be gripped by the hand to enable the outer casing 30 to be slid axially relative to the inner element 20.

Inside the collection chamber R there is positioned a longitudinal rod 40 of lesser cross-section than the cross-section of the collection chamber R, which extends longitudinally through the chamber R, and with its rear end fixed to the inner element 20.

The scraper blade 50 is fixed to the front end of the rod 40 and is disposed to the front of the front end portion of the outer casing 30.

The scraper blade 50 and the longitudinal rod 40 are independent of the outer casing 30, at least part of the rod being spaced in a radial direction from the inner surface of the outer casing 30 so that it can flex with a relative movement towards this surface when the blade, when in use, is urged against the bone in a radial direction.

The longitudinal rod 40 is of such dimensions and shape as to make it, in relation to its composition material, elastically deformable so that it moves in a direction radial to the edge of the front end portion 31 of the outer casing when the scraping edge 51 of the blade 50, when in use, is urged against the bone in a radial direction.

In a preferred (but not exclusive) embodiment of the scraper blade 50, this presents a scraping edge 51, for effecting the bone scraping action, which lies in a plane virtually perpendicular to the axis of the outer casing 30 (see Figure 3) or inclined thereto (see Figure 10).

Specifically, the scraper blade 50 is of mushroom shape having a slightly conical front portion 52 joined to a relatively thin shank 54 fixed to the longitudinal rod 40; the front portion 52 has a flat or virtually flat rear face 53 which extends radially from the shank 54, its lower portion forming a scraping edge 51 for scraping the bone.

The rear face 53 of the blade 50 adheres to the edge of the front end portion 31 at the collection chamber R to close the front end of the chamber. However, at the scraping edge 51 of the blade, the edge of the front end portion 31 possesses a part 31 a in the form of a small-length arc lying a certain distance from the scraping edge 51 to define, in combination with the rear face 53 of the scraping edge 51, a narrow slit F enabling the bone flakes or shavings to pass to the collection chamber R. In a first embodiment, shown in the figures, the front portion 52 of the blade 50 is in the form of a rounded cone or ogive, having a central axis A of symmetry coinciding with the axis of the longitudinal rod 40 to which it is fixed; it also has a flat or virtually flat major base perpendicular to the axis A and defining the rear face 53 of the blade, of which the lower part defines said scraping edge 51.

The blade 50 also has a maximum outer dimension (the major base of the cone frustum) which is virtually equal, and preferably exactly equal, to the cross-section of the front end portion 31 of the outer casing 30 and is positioned to close this latter; it is also disposed out of alignment with the front end portion 31 so that the scraping edge 51 of the blade projects slightly outwards in a radial direction beyond the outer profile of said front end portion 31.

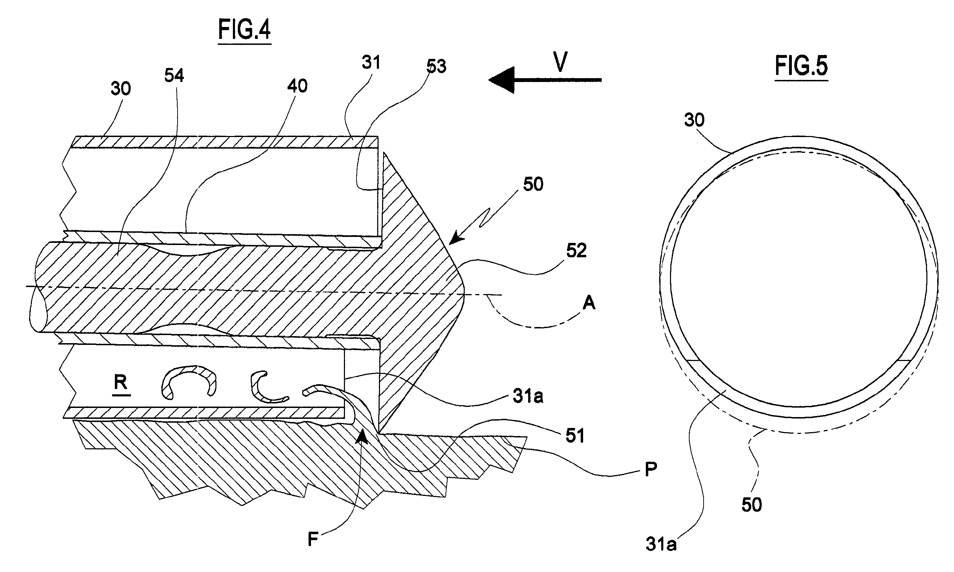

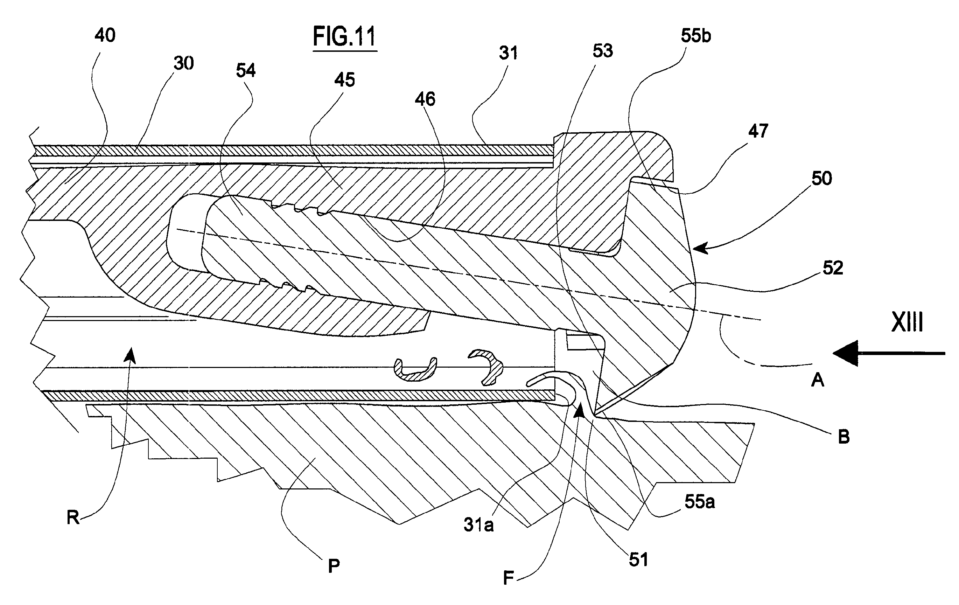

When in use, the operator's hand grips the handle of the instrument in correspondence with the outer casing 30 then, after positioning the axis of the instrument virtually parallel or inclined to the bone surface, presses the blade radially against the bone; because of the elasticity of the rod 40, the blade 50 withdraws radially from the outer casing 30; when in this position the scraping edge 51 projects further forwards, by a gauged amount of a few tenths of a millimetre, from the outer surface of the front end portion 31 (see Figures 4 and 11). Then on scraping, while the front end portion 31 is held in contact with the bone, the scraping edge 51 penetrates into the bone tissue for a gauged and precise distance with a (constant) thrust determined by the physical characteristics of the instrument; by virtue of this, the transverse scraping thrust is controlled not only by the operator but also by the instrument characteristics, with the result that the operation is better controllable.

**In the first embodiment**, the longitudinal rod 40 is joined to the inner element 20 along the axis thereof and is slightly inclined to that axis so that its front end, and with it the blade 50, are out of alignment with the front end portion 31 of the outer casing. The scraping edge 51 of the blade is thus made to project slightly outwards radially by a small distance (from 0.05 to 0.3 mm) from the outer profile of the front end portion 31 (Figure 4).

The inner element 20 has a cylindrical surface and a constant circular cross-section at the front end portion 21 and also at other portions of the remaining part; likewise the casing 30 has a constant circular cross-section and can be advantageously manufactured by cutting segments from a commercially obtainable stainless steel cannula for laparoscopic use having an outer diameter of 5-6 mm and a thickness of 0.2 mm.

The longitudinal rod 40 also consists of an internally empty thin cannula, in particular of circular cross-section, its rear end portion being fixed axially by insertion into a seat 22 provided in the front end portion 21 of the inner element 20, the blade shank 54 being substantially perpendicular to the scraping rear face 53 of the blade and being fixed by forced insertion into the front end portion of the cannula.

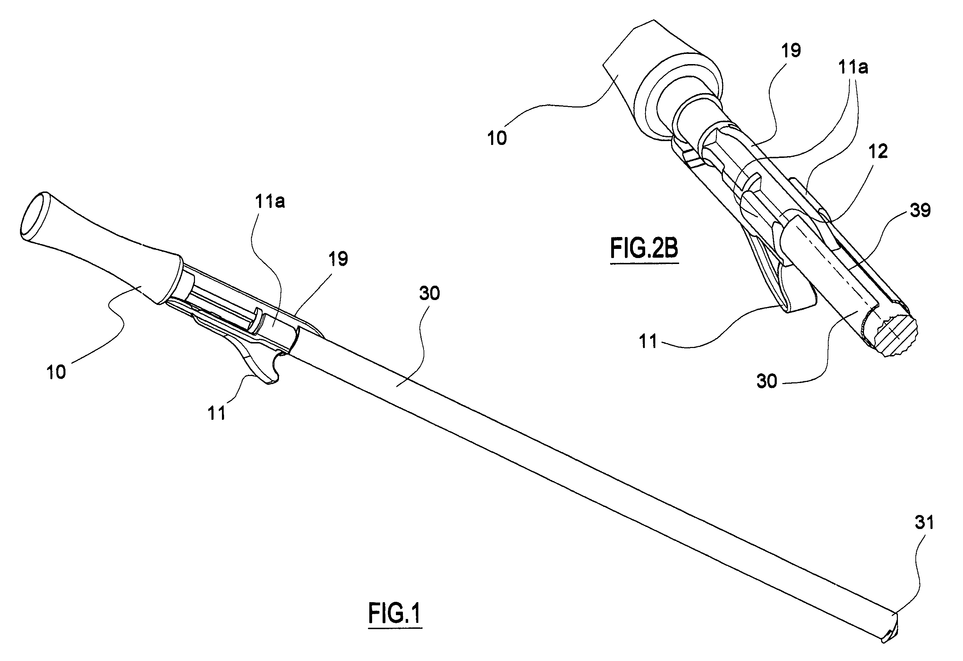

To prevent rotation between the outer casing 30 and the inner element 20, there is provided for example a constant-thickness longitudinal rib 19, which projects in radial relief from the rear handgrip 10, and engages a longitudinal slot 39 provided in the front end portion of the outer casing 30. A manually operable pusher 11 is connected to the handgrip 10 to abut against the edge of the rear end of the casing 30, to maintain it locked in the front end position. The pusher 11 possesses a front end 11 a in the form of a fork which snap-embraces a seat 12 provided in the lateral surface of the handgrip 10 so as to be secured to it with a certain force; this front end 11 a abuts against the front end of the casing 30, located in a front position, to prevent this being able to be pulled rearwards (see Figure 2). To pull the casing 30 rearwards and hence open the collection chamber R, the pusher 11 must firstly be manually displaced from the seat 12, to release the casing 30 (see Figure 2A).

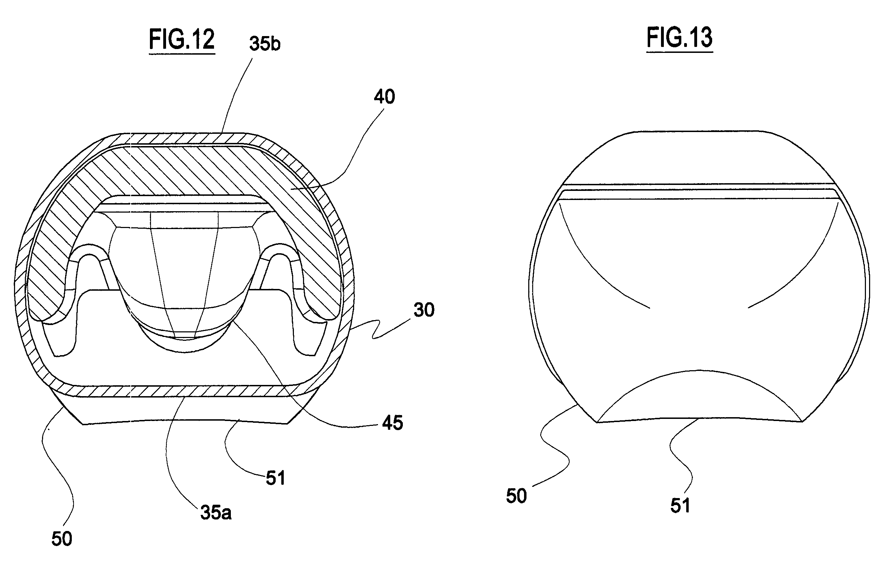

**In the second embodiment**, the casing 30 is of constant cross-section with a profile in the form of a circle cut by at least one surface forming a lower flat side 35a (see Figure 12) positioned at the scraping edge 51; at least the front end portion 21 of the inner element 20 also has an identical profile, to adhere to the inner surface of the outer casing.

The presence of said lower side 35a increases, when in use, the general adherence of the instrument to that surface of the bone tissue to be operated on, making sliding more secure and regular.

The profile of the outer casing 30 and of the inner element 20 also comprise a second upper flat side 35b, opposite the lower side 35a. This serves to reduce the vertical transverse dimension of the instrument to facilitate its penetration along the boundary surface between two adhering tissues.

The front portion 52 of the blade 50 is overall of approximately conical or cap shape having, at the scraping edge 51, a lower region 55a cut as a flat or slightly concave surface with generators inclined to the blade axis A; the scraping edge 51 is defined by the intersection between said lower region 55a and the rear face of the blade and presents a profile in the form of a more or less concave arc.

This blade is suitable for operating on convex curved surfaces having substantially parallel generators, typically in rhynoplasty to remove material from nasal bones.

In contrast to the first embodiment, the longitudinal rod 40 is of complex form, its rear portion 44 being positioned adhering to the inner surface of the outer casing 30. Its front end portion 45 is positioned at a distance, in the radial direction, from the inner surface of the casing 30 so that it can flex in a radial direction towards it when, while in use, the blade is urged against the bone in a radial direction. The cross-section of the rod 40 is of overall curved shape, bounded by a convex surface adhering to the inner surface of the outer casing 30 and by a concave surface facing the collection chamber R. Because of these characteristics, the space occupied by the rod 40 within the collection chamber R is a minimum and consequently its useful cross-section becomes a maximum, this facilitating general miniaturization of the instrument.

The front end portion 45 of the rod presents a widening of thickness suitable for forming a seat 46 into which the shank 54 of the blade is forcibly inserted. The blade 50 is disposed such that the scraping edge 51 slightly projects radially outwards from the outer profile of the front end portion 31 of the outer casing 30. In this case, the shank 54 has a substantial inclination (10-15 degrees) to the longitudinal axis of the outer casing 30, so that the rake angle (defined between the rear face 53 and the straight line perpendicular to the bone surface) has a substantial value greater than zero.

The front portion 52 of the blade 50 possesses a flat upper face 55b to rest or nearly rest against a corresponding seat 47 provided on the front end portion 45, with the double purpose of acting as a guide when connecting the blade 50 to the seat 46, and preventing the blade 50 oscillating about the axis of its shank 54.

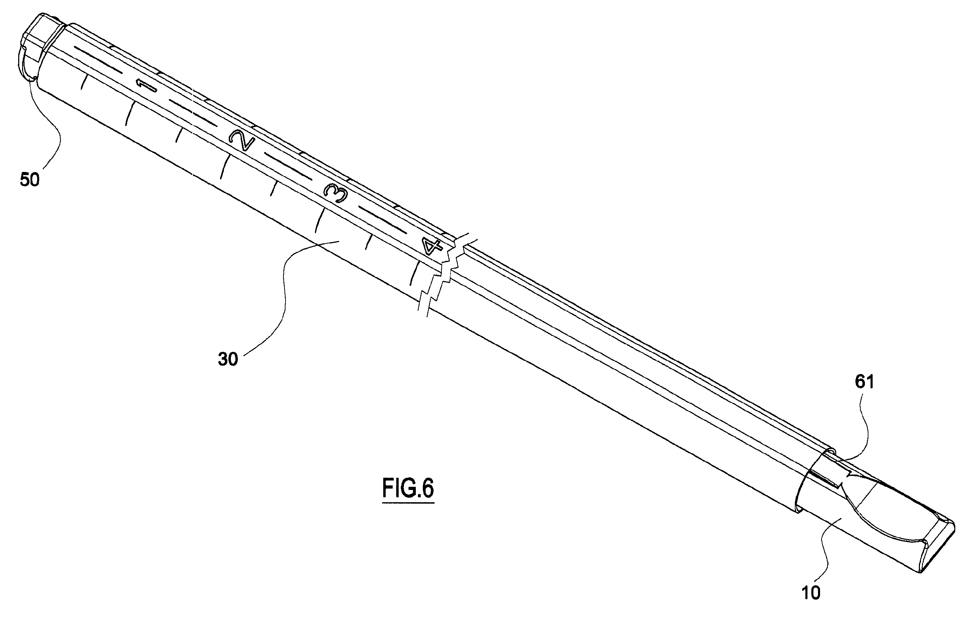

A manually operable tooth 61 is provided on the handgrip 10 to abut against the edge of the front end of the casing 30 to maintain it locked in the front end portion. Specifically, the tooth 61 acts against the rear end of the casing 30, when in its front position, to prevent this being able to be pulled rearwards (see Figures 6 and 9). To pull the casing 30 rearwards and hence open the collection chamber R, the tooth 61 must firstly be moved manually to flex the rear portion of the handgrip 10 in order to release the casing 30.

Finally, as in the first embodiment, a constant-thickness longitudinal rib 19 is provided, which projects in radial relief from the rear handgrip 10, and engages a longitudinal slot 39 provided in the front end portion of the outer casing 30.

Numerous modifications of a practical and applicational nature can be made to the invention, but without leaving the scope of the inventive idea as claimed below.

## Claims

1. An instrument for removing and/or collecting flakes or shavings from bone by scraping, comprising a handle carrying a scraper blade (50) positioned at its front end, and possessing in its front end portion a collection chamber (R) for the removed material,
**characterised in that** the handle comprises:
an elongate inner element (20), having at least a front portion (21) with a cylindrical surface,
a tubular outer casing (30) which encloses the inner element (20) about its entire perimeter and adheres to the outer surface of its front portion (21),
a longitudinal rod (40) having a cross-section less than the cross-section of the collection chamber (R) to extend longitudinally along and within the chamber, its rear end being fixed to the inner element (20),
the scraper blade (50) being fixed to the front end of the longitudinal rod (40).

2. An instrument as claimed in claim 1, **characterised in that** said outer casing (30) is slidable in a longitudinal direction relative to the inner element (20) between a front position in which its front end portion (31) projects forwards longitudinally to a maximum extent from the front portion (21) of the inner element (20), to define said collection chamber (R), and a rear position in which the outer casing (30) is displaced rearwards to project from the inner element to a lesser extent than in the front position, the collection chamber (R) being at least partly open.

3. An instrument as claimed in claim 1, **characterised in that** the scraper blade (50) and the longitudinal rod (40) are independent of the outer casing (30), at least part of the rod (40) being spaced in a radial direction from the inner surface of the outer casing (30) so that it can flex towards said surface in a radial direction when the blade (50), when in use, is urged against the bone in a radial direction.

4. An instrument as claimed in claim 3, **characterised in that** the longitudinal rod (40) is of such dimensions and shape, in relation to its composition material, as to make it elastically deformable so that it moves in a direction radial to the front edge of the outer casing (30) when the blade (50), when in use, is urged against the bone in a radial direction.

5. An instrument as claimed in claim 1, **characterised in that** the rear face (53) of the blade (50) closes the front end of the collection chamber (R) by adhering to the edge of the front end portion (31) of the casing (30), the edge of the front end portion (31) possessing at the blade scraping edge (51) a part (31 a) lying at an axial distance from the scraping edge (51) to define, in combination with the rear face (53) of the scraping edge (51), a narrow slit (F) enabling the bone flakes or shavings to pass to the collection chamber (R).

6. An instrument as claimed in claim 1, **characterised in that** the scraper blade (50) has a maximum outer dimension substantially equal to the cross-section of the front end portion (31) of the outer casing (30) and is disposed out of alignment therewith so that its scraping edge (51) projects slightly outwards radially beyond the outer profile of the front end portion (31) of the outer casing (30).

7. An instrument as claimed in claim 5, **characterised in that** the longitudinal rod (40) is joined to the inner element (20) along the axis thereof and is slightly inclined to that axis so that its front end, and with it the blade (50), are out of alignment with the front end portion (31) of the outer casing (30).

8. An instrument as claimed in claim 1, **characterised in that** the inner element (20) has a cylindrical surface and a constant circular cross-section at at least the front portion (21), the casing 30 likewise having a constant circular cross-section

9. An instrument as claimed in claim 1, **characterised in that** the scraper blade (50) is of mushroom shape having a slightly pointed front portion (52) and, fixed to the longitudinal rod (40), a relatively thin shank (54) joined to rear face (53) of the front portion, said rear face (53) being flat or virtually flat, its lower portion forming a cutting edge (51) for scraping the bone.

10. An instrument as claimed in claim 1, **characterised in that** the longitudinal rod (40) consists of an internally empty thin cannula, the rear end portion of which is fixed axially by insertion into a seat (22) provided in the front end portion (21) of the inner element (20), the scraper blade (50) comprising a shank (54) which is fixed by forced insertion into the front end portion of the cannula.

11. An instrument as claimed in claim 1, **characterised in that** the handle comprises a rear handgrip (10), to which the inner element (20) is joined and which remains open with respect to the outer casing (30), to be gripped by the hand in order to slide the outer casing (30) relative to the inner element (20).

12. An instrument as claimed in claim 1, **characterised in that** the casing (30) is of constant cross-section with a profile in the form of a circle cut along at least one flat side (35a), at least the front portion (21) of the inner element (20) having an identical profile to adhere to the inner surface of the outer casing (30), said flat said (35a) being disposed in correspondence with the scraping edge (51).

13. An instrument as claimed in claim 9, **characterised in that** the front portion (52) of the blade (50) is overall of approximately conical or cap shape having, at the scraping edge (51), a lower region (55a) cut as a flat or slightly concave surface with generators inclined to the blade axis A, the scraping edge (51) being defined by the intersection between said lower region (55a) and the rear face (53) of the blade.

14. An instrument as claimed in claim 9, **characterised in that** the front portion (52) of the blade (50) possesses, opposite the scraping edge (51), a flat upper face (55b) to rest against a corresponding seat (47) provided on the front end portion (45) of the rod (40).

15. An instrument as claimed in claim 3, **characterised in that** the longitudinal rod (40) is disposed with its rear portion adhering to the inner surface of the outer casing (30), its front end portion being positioned at a distance, in the radial direction, from said inner surface so that it can flex in a radial direction towards it when, while in use, the blade (50) is urged against the bone in a radial direction.

16. An instrument as claimed in claim 13, **characterised in that** the cross-section of the rod (40) is of overall curved shape, bounded by a convex surface adhering to the inner surface of the outer casing (30) and by a concave surface facing the collection chamber R.
